# EUROPEAN PATENT SPECIFICATION

(11) **EP 3 810 245 B1**
(45) Date of publication and mention of the grant of the patent: **18.11.2026**
(21) Application number: 19821731.7
(22) Date of filing: 21.06.2019
(51) Int. Cl.: A61L 29/14, A61M 39/20, A61M 39/16, A61L 31/16, A61L 29/16, A61M 25/00

(54) **SYSTEM FOR STERILIZING INTRAVENOUS CONNECTORS AND TUBING**
SYSTEM ZUR STERILISATION VON INTRAVENÖSEN VERBINDERN UND SCHLÄUCHEN
SYSTÈME DE STÉRILISATION DE RACCORDS ET DE TUBULURES INTRAVEINEUSES

(30) Priority: 21.06.2018 US 201862688203 P
(43) Date of publication of application: 28.04.2021
(73) Proprietor: ICU Medical, Inc., San Clemente, CA 92673 (US)
(72) Inventor: FANGROW, Thomas, F., San Clemente, CA 92673 (US)
(74) Representative: Reddie & Grose LLP
(86) International application number: PCT/US2019/038351
(87) International publication number: WO 2019/246472

(56) References cited:
- WO-A1-2014/074929
- US-A1- 2005 124 970
- US-A1- 2005 124 970
- US-A1- 2009 062 766
- US-A1- 2009 062 766
- US-A1- 2013 144 258
- US-A1- 2013 144 258
- US-A1- 2013 150 795
- US-A1- 2016 001 056
- US-A1- 2016 101 276

## Description

### PRIORITY CLAIM

This application claims priority to U.S. Provisional Patent Application No. 62/688,203, filed on June 21, 2018.

### BACKGROUND OF THE DISCLOSURE

### Field of the Disclosure

The present disclosure relates in general to the field of medical connectors and tubing, and in particular to therapeutic delivery devices for use with such medical connectors and tubing.

### Description of the Related Art

The preparation and administration of fluids in hospitals and medical settings routinely involves the use of medical connectors and catheters. Needleless connectors are typically structured so that a medical implement without a needle can be selectively connected to such a connector for providing fluid flow between a patient and a fluid source or receptacle. When the medical implement is removed, the medical connector closes, effectively sealing the connection to the patient without requiring multiple injections to the patient and without exposing health care professionals to the risk of inadvertent needle sticks. The medical implement used with the connector can be a tube or other medical device such as a conduit, syringe, IV set (both peripheral and central lines), piggyback line, or similar component which is adapted for connection to a medical valve.

A catheter that provides access to a patient's vasculature can be connected to a distal end of a medical connector such as described above. A proximal end of the medical connector can be connected to a medical implement such as described above. The catheters that provide access to a patient's vasculature (for example, hemodialysis catheters) can remain in a blood vessel for an extended period of time (for example, about 2-3 days to about a week, or much longer). When the medical implement is disconnected, some devices or fluid administration techniques have attempted to keep microbes from entering the patient's bloodstream via the catheter or via the medical connector. Microbes in the bloodstream can increase the risk of blood infections, commonly known as catheter-related bloodstream infections (CRBSIs), in the patient. There are many negative effects of CRBSI's, including serious health risks and increased costs for additional patient treatment. It is common practice in situations where the risk of contracting a CRBSI is high, such as in long-term uses of catheters, to provide a static antimicrobial locking solution in the catheter when fluid is not being transferring to or from the patient through the catheter. US 2005/0124970 A1 describes a luer fitting. The luer fitting includes a body having a first end and a second end, wherein the first end comprises an antibacterial component disposed therewith. The luer fitting is connected over a catheter that has been primed with a locking solution. The antibacterial component is fluidly engaged with the locking solution such that the antibacterial component diffuses through the locking solution. A method of reducing antibacterial activity in a catheter utilizing the fitting is also described. WO 2014/074929 describes a medical connector for use in a fluid pathway including a housing configured to permit fluid flow between a first medical device and a second device or location. The medical connector includes a valve member configured to be positioned at least partially within the housing. The valve member is configured to receive the first medical device. The valve member can be moved into a second state. The connector can be adapted for use with a catheter assembly. US 2013/0144258 describes systems, methods, and articles for delivering an antimicrobial composition into the lumen of a trans-dermal catheter and to proximal elements of the transdermal catheter are disclosed. In an embodiment, the system comprises an elongate member configured for insertion into a lumen of a catheter, the elongate member containing an antimicrobial. In an alternative embodiment, an antimicrobial composition placed on an interior surface a cap element, and transfers antimicrobial to the exterior of the proximal end of the trans-dermal catheter. US 2016/101276 A1 discusses flush syringe assemblies comprising an integrated contamination-prevention device integrated with device connector flushing positioned so that the practitioner cannot forget to apply disinfectant. The flush syringe assemblies comprise a barrel with an elongate plunger rod disposed therein and a cap comprising a passageway. The plunger rod includes a stopper of which at least a portion can be embedded in the passageway of the cap to form a plug in the cap.

### SUMMARY

The invention is defined in the appended independent claims, to which attention is directed. Optional features are set out in the dependent claims. Disclosed are embodiments of a therapeutic delivery device for medical connectors and tubing and examples of methods for performing an antimicrobial lock procedure. In some embodiments, a therapeutic delivery device is used in performing an antimicrobial lock procedure with a medical fluid connector having a seal with a normally closed slit. The therapeutic delivery device can include a head portion configured to engage a proximal end of a medical fluid connector; a distally extending elongate shaft configured to be inserted into a valve member of the medical fluid connector, wherein one or more antimicrobial materials are disposed on at least a portion of the elongate shaft and are configured to be in contact with a fluid in a catheter coupled to the medical fluid connector; and a cylindrical base portion connecting a distally facing surface of the head portion and a proximal end of the elongate shaft, the cylindrical base portion being configured to be positioned outside of the slit when the therapeutic delivery device is fully attached to the medical fluid connector. In some embodiments, the elongate shaft of the therapeutic delivery device has one or more portions with an uneven or non-smooth surface. In some embodiments, the one or more antimicrobial materials are disposed on one or more portions of the therapeutic delivery device, such as on one or more portions of the elongate shaft (e.g., in one or more recesses, or between protrusions, and/or on the majority or substantially all of the shaft), on one or more portions of the head portion (e.g., on an outside surface, on an underside, on an inside surface, and/or on threading, if present, etc.), on a base portion, and/or all or substantially all of the outer surface of the therapeutic delivery device.

In some embodiments, a method of sterilizing a catheter and a valve member can use a therapeutic delivery device. The catheter can comprise a lumen between a distal end and a proximal end of the catheter. The valve member can comprise a slit that is biased to a closed position. The distal end of the catheter can be configured to be located within a patient and the proximal end of the catheter configured to be located outside of the patient, the valve member configured to be coupled with the proximal end of the catheter. The method can comprise injecting a fluid into the lumen of the catheter; providing a therapeutic delivery device with a base portion, an outer diameter of the base portion being larger than a length of the slit on the valve member; inserting the therapeutic delivery device through the slit on the valve member so that the therapeutic delivery device is in fluid communication with the fluid in the lumen of the catheter, the therapeutic delivery device comprising one or more antimicrobial materials disposed on at least a portion of the therapeutic delivery device that are placed in fluid communication with the fluid; and forming a seal by contact between the base portion and the valve member at the slit, the one or more antimicrobial materials configured to be in contact with and released into the fluid to form an antimicrobial locking solution.

In some embodiments, the method can further comprise applying a clamp across a portion of the catheter outside the patient after the inserting, the clamp substantially preventing fluidic communication between portions of the catheter proximal and distal of the clamp. In some embodiments, the therapeutic delivery device can comprise an elongate shaft extending distally from the base portion, the one or more antimicrobial materials disposed on at least a portion of the elongate shaft. In some embodiments, when assembled, at least the portion of the elongate shaft on which the one or more antimicrobial materials are disposed can extend into the lumen of the catheter. In some embodiments, the inserting can comprise opening the slit using a distal end of the elongate shaft. In some embodiments, the at least a portion of the elongate shaft on which the one or more antimicrobial materials are disposed can comprise an uneven surface and/or one or more cavities. In some embodiments, the valve member can be located in a medical fluid connector having a proximal end and a distal end, the distal end of the medical fluid connector coupled to the proximal end of the catheter. In some embodiments, the therapeutic device can comprise a head portion, and wherein the forming the seal can further comprise engaging the head portion of the therapeutic delivery device with the proximal end of the medical fluid connector so as to press the base portion into contact with the valve member at the slit. In some embodiments, the medical fluid connector can comprise a non-tortuous fluid path. In some embodiments, the injecting can comprise using a medical implement coupled to the proximal end of the medical fluid connector, the medical implement being de-coupled from the medical fluid connector prior to the inserting of the therapeutic delivery device, the medical fluid connector comprising a fluid path having an internal diameter that is substantially the same as an outlet port of the medical implement. In some embodiments, the medical fluid connector can be a needle-less connector. In some embodiments, the medical fluid connector can be spike-less. In some embodiments, the base portion can be configured to be positioned proximal and outside of the slit when the therapeutic delivery device is fully attached to the medical fluid connector. In some embodiments, the valve member can comprise a more deformable and/or more resilient material than the base portion such that the valve member is configured to conform around a distal and/or side surface of the base portion to form the seal. In some embodiments, the one or more antimicrobial materials can be disposed on substantially an entire surface of the therapeutic delivery device.

In some embodiments, a method can be used to sterilize a catheter. The catheter can comprise a lumen between a distal end and a proximal end, the distal end of the catheter configured to be located within a patient and the proximal end of the catheter configured to be located outside of the patient. The method can comprise coupling a selectively resealable valve member to the proximal end of the catheter, the lumen of the catheter configured to contain a fluid; and providing a therapeutic delivery device configured to be inserted through an opening on the valve member so that the therapeutic delivery device can be positioned in fluid communication with the fluid in the lumen of the catheter, the opening being initially closed to seal the proximal end of the catheter, the therapeutic delivery device comprising one or more antimicrobial materials that can be configured to be in fluid communication with the fluid, the therapeutic delivery device further comprising a base portion, an outer diameter of the base portion being larger than a diameter of the opening. When assembled, the one or more antimicrobial materials can be configured to be in contact with and released into the fluid to form an antimicrobial locking solution.

In some embodiments, the therapeutic delivery device can comprise an elongate shaft extending distally from the base portion, the one or more antimicrobial materials disposed on at least a portion of the elongate shaft. In some embodiments, when assembled, at least the portion of the elongate shaft on which the one or more antimicrobial materials are disposed can extend into the lumen of the catheter. In some embodiments, a distal end of the elongate shaft can be configured to open the opening. In some embodiments, the at least a portion of the elongate shaft on which the one or more antimicrobial materials are disposed can comprise an uneven surface and/or one or more cavities. In some embodiments, the one or more antimicrobial materials can be disposed on substantially an entire surface of the therapeutic delivery device. In some embodiments, providing the valve member can comprise providing a medical connector containing the valve member, the medical connector having a proximal end and a distal end, the distal end of the medical connector coupled to the proximal end of the catheter. In some embodiments, when assembled, a head portion of the therapeutic device can engage the proximal end of the medical fluid connector so as to press the base portion into contact with the valve member at the opening to form the seal. In some embodiments, the base portion can be configured to be positioned proximal and outside of the opening when the therapeutic delivery device is fully attached to the medical fluid connector. In some embodiments, the medical fluid connector can comprise a non-tortuous fluid path. In some embodiments, the medical fluid connector can be spike-less. In some embodiments, the fluid in the lumen of the catheter can be injected using a medical implement coupled to the proximal end of the medical fluid connector, the medical implement being de-coupled from the medical fluid connector prior to insertion of the therapeutic delivery device, the medical fluid connector comprising a fluid path having an internal diameter that is substantially the same as an outlet port of the medical implement. In some embodiments, the medical fluid connector can be a needle-less connector. In some embodiments, the valve member can comprise a more deformable and/or more resilient material than the base portion such that the valve member is configured to conform around a distal and/or side surface of the base portion to form the seal.

A therapeutic delivery system for use in performing an antimicrobial lock procedure comprises a medical fluid connector having a proximal end and a distal end. The system also comprises a selectively resealable valve member comprising a normally closed slit. The valve member is configured to seal a proximal end of a catheter, the selectively resealable valve member is located in the medical fluid connector having the proximal end and the distal end. The system further comprises a therapeutic delivery device including an elongate shaft and a base portion, the elongate shaft extends distally of the base portion, an outer diameter of the base portion being greater than a length of the slit. One or more antimicrobial materials are disposed on at least the elongate shaft, wherein the elongate shaft is configured to be inserted through the slit and the one or more antimicrobial materials are disposed at least partially on an outer shaft surface of the shaft inserted through the slit. When assembled, contact between the base portion and the valve member at the slit forms a seal and the one or more antimicrobial materials are configured to be in contact with and released into a fluid in the catheter to form an antimicrobial locking solution.

In some embodiments, the medical fluid connector can comprise a non-tortuous fluid path. In some embodiments, the medical fluid connector can comprise a fluid path having an internal diameter that is substantially the same as an outlet port of a medical implement used to inject the fluid into the catheter. In some embodiments, the valve member can be located at or near a proximal end of the medical fluid connector. In some embodiments, the therapeutic delivery device can comprise a head portion configured to engage a proximal end of the medical fluid connector so as to press the base portion into contact with the valve member at the slit to form the seal. In some embodiments, the medical fluid connector can be spike-less. In some embodiments, the base portion can be configured to be positioned proximal and outside of the slit when the therapeutic delivery device is fully attached to the medical fluid connector. In some embodiments, the medical fluid connector can be a needle-less connector. In some embodiments, a distal end of the elongate shaft can be configured to open the slit. In some embodiments, the at least a portion of the elongate shaft with the one or more antimicrobial materials are disposed can comprise an uneven surface and/or one or more cavities. In some embodiments, the one or more antimicrobial materials can be disposed on substantially an entire surface of the therapeutic delivery device. In some embodiments, the valve member can comprise a more deformable and/or more resilient material than the base portion such that the valve member can be configured to conform around a distal and/or side surface of the base portion to form the seal.

In some embodiments, a locking system for applying an antimicrobial locking solution to a catheter has a lumen between a distal end and a proximal end, the distal end of the catheter configured to be located within a patient and a proximal end configured to be located outside of the patient. The locking system can include: a medical connector having a proximal end and a distal end, the proximal end of the catheter releasably coupled to the distal end of a medical connector, the medical connector comprising a valve with a slit at the proximal end of the medical connector, the slit being normally closed to create a seal to the proximal end of the catheter; and a therapeutic delivery device configured to be inserted into the medical connector, the therapeutic delivery device comprising a head portion and a distally extending elongate shaft, wherein one or more antimicrobial materials are disposed on at least a portion of the elongate shaft, a distal end of the distally extending elongate shaft configured to open the slit to enter the medical connector, the therapeutic delivery device further comprising a base portion connecting a distally facing surface of the head portion and a proximal end of the elongate shaft, an outer diameter of the base portion being greater than a length of the slit.

In some embodiments, when assembled, the distal end of the distally extending elongate shaft extends distally from the distal end of the medical device and the head portion engages the proximal end of the medical connector such that contact between the base portion and the valve member at the slit forms a seal, the antimicrobial material(s) configured to be in contact with and released into the injected fluid to form an antimicrobial locking solution. In some embodiments, at least a portion of the elongate shaft with the one or more antimicrobial materials comprises an uneven surface. In some embodiments, the one or more antimicrobial materials are disposed on an entire surface of the therapeutic delivery device. In some embodiments, the system can include a clamp configured to be applied across a portion of the catheter outside the patient, the clamp substantially preventing fluidic communication between portions of the catheter proximal and distal of the clamp.

In some embodiments, a method of applying an antimicrobial locking solution to a catheter using a therapeutic delivery device can include: (a) injecting a fluid into a lumen of the catheter, wherein a distal end of the catheter is located within a patient and a proximal end of the catheter is located outside of the patient, the proximal end of the catheter coupled to a distal end of a medical connector, the medical connector comprising a valve with a slit at a proximal end of the medical connector, the slit being closed to create a seal to the proximal end of the catheter; (b) inserting the therapeutic delivery device into the medical connector, the therapeutic delivery device comprising a head portion and a distally extending elongate shaft, wherein one or more antimicrobial materials are disposed on at least a portion of the elongate shaft, a distal end of the elongate shaft configured to open the slit to enter the medical connector, the therapeutic delivery device further comprising a base portion connecting a distally facing surface of the head portion and a proximal end of the elongate shaft, an outer diameter of the base portion being greater than a length of the slit, wherein when assembled, the distal end of the distally extending elongate shaft extends distally from the distal end of the medical connector and the head portion engages the proximal end of the medical connector such that contact between the base portion and the valve member at the slit forms a seal, the one or more antimicrobial materials configured to be in contact with and released into the injected fluid to form an antimicrobial locking solution; and (c) applying a clamp across a portion of the catheter outside the patient before or after insertion of the therapeutic delivery device into the medical connector, the clamp substantially preventing fluidic communication between portions of the catheter proximal and distal of the clamp. In some embodiments of the method, at least a portion of the elongate shaft with the one or more antimicrobial materials comprises an uneven surface. In some embodiments, the one or more antimicrobial materials are disposed on an entire surface of the therapeutic delivery device.

### BRIEF DESCRIPTION OF THE DRAWINGS

These and other features, aspects, and advantages of the present disclosure are described with reference to the drawings of certain embodiments, which are intended to schematically illustrate certain embodiments and not to limit the disclosure.
Figure 1A illustrates a top perspective view of an example of a therapeutic delivery device.
Figure 1B illustrates a bottom perspective view of the therapeutic delivery device of Figure 1A.
Figure 1C illustrates a top view of the therapeutic delivery device of Figure 1A.
Figure 1D illustrates a bottom view of the therapeutic delivery device of Figure 1A.
Figure 1E illustrates a front view of the therapeutic delivery device of Figure 1A.
Figure 1F illustrates a side view of the therapeutic delivery device of Figure 1A.
Figure 2A illustrates a cross-sectional view of the therapeutic delivery device of Figure 1E along the axis 2A-2A.
Figure 2B illustrates a cross-sectional view of the therapeutic delivery device of Figure 1F along the axis 2B-2B.
Figure 3A illustrates an exploded view of an assembly of an example therapeutic delivery device and an example medical connector.
Figure 3B illustrates a cross-sectional view of the medical connector of Figure 3A along the axis 3B-3B.
Figure 3C illustrates a longitudinal cross-sectional view of an assembly of an example therapeutic delivery device partially inserted into an example medical connector.
Figure 3D illustrates a longitudinal cross-sectional view of the assembly of Figure 3C, with the therapeutic delivery device substantially fully inserted into the medical connector in some embodiments.
Figure 3E illustrates a longitudinal cross-sectional view of the assembly of Figure 3C, with the therapeutic delivery device substantially fully inserted into the medical connector in some embodiments.

### DETAILED DESCRIPTION

Although certain embodiments and examples are described below, those of skill in the art will appreciate that the disclosure extends beyond the specifically disclosed embodiments and uses. Thus, it is intended that the scope of this disclosure should not be limited by any particular embodiments described below.

Conventional procedures for attempting antimicrobial locks are time-consuming, require the acquisition, storage, and use of multiple liquids, and may not deliver the anti-microbial solution in an effective dosage or in a useful timing sequence. Some embodiments disclosed herein address one or more of these issues and/or other issues that can occur when using a catheter or connector or while performing a conventional antimicrobial lock method with conventional equipment. In some embodiments, a therapeutic delivery device is configured to provide one or more therapeutic agents (for example, antimicrobial agents, anticlotting agents, or others) to a fluid (for example, water, saline, heparin, or others) in the catheter to form a locking solution. In some embodiments, the therapeutic delivery device can include coating(s) of the one or more therapeutic agents on a portion of the device that can come into contact with the fluid inside the catheter. The coating of one or more therapeutic agents can be released into the fluid to provide a locking solution. In some embodiments, one or more portion(s) of the therapeutic delivery device coated with the one or more therapeutic agents can have an uneven or non-smooth surface to reduce wipe-off of the coated one or more therapeutic agents when introducing the therapeutic delivery device into a medical connector coupled to the catheter. In some embodiments, fluid communication between a proximal portion of the catheter and a remainder of the catheter can be suspended (for example, with use of a tubing clamp, a clip, or other mechanisms for temporarily stopping fluid flow in a catheter) so that the locking solution remains in the proximal portion of the catheter.

### Examples of Therapeutic Delivery Devices

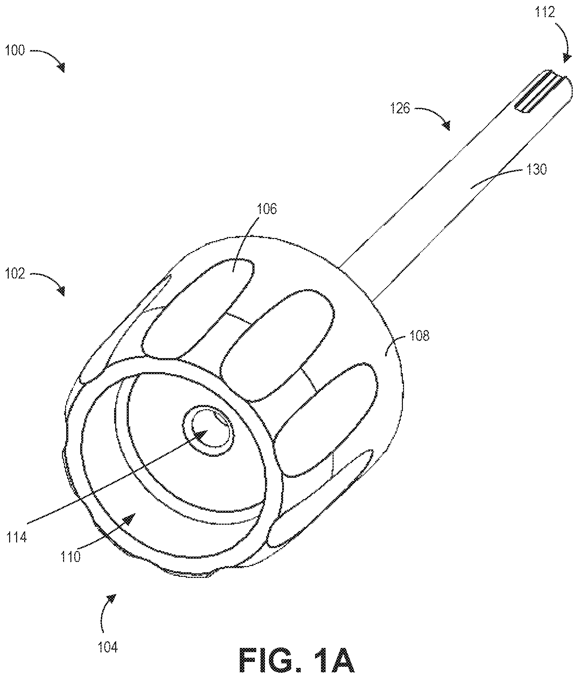

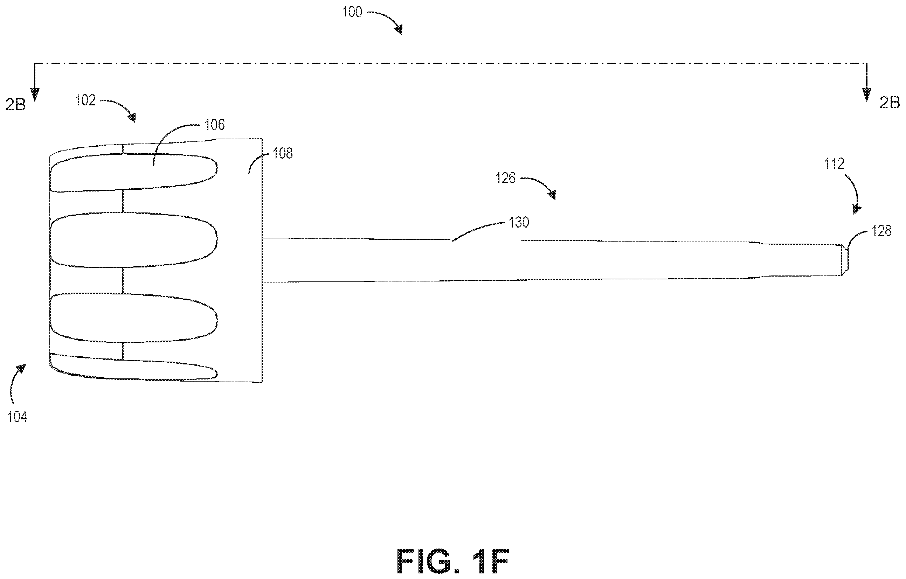

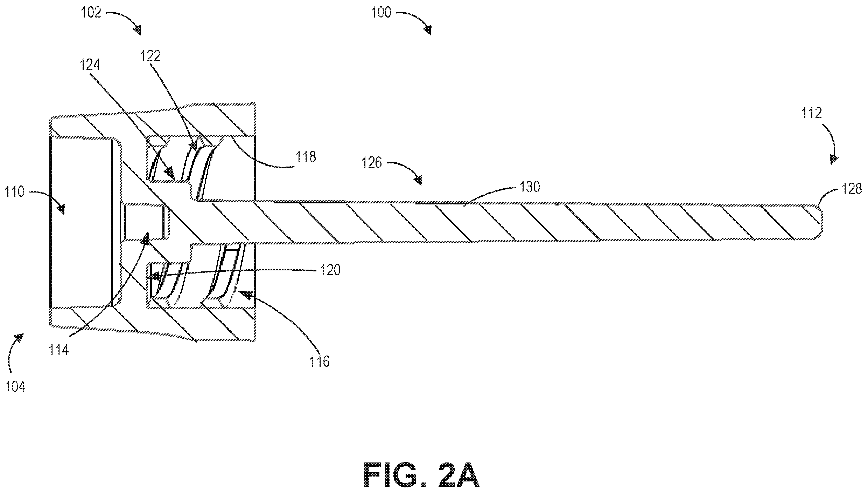

With reference to the figures, certain embodiments and examples of therapeutic delivery devices for use with medical connectors will now be described. As used herein, "proximal" refers to the end or direction that is closest or closer to a clinician working with the therapeutic delivery device. As illustrated, in some situations, proximal is analogous to "top" as shown with respect to Figures 1A-1F and 2A-2B.

Figures 1A-1F and 2A-2B illustrate an example therapeutic delivery device 100 for use with a medical connector (for example, in providing increased antimicrobial resistance or in performing an antimicrobial lock procedure). The therapeutic delivery device 100 can include a head portion 102 at a proximal end 104 of the device 100. The head portion 102 can include a generally cylindrical outer shape. In other embodiments, the head portion 102 can have an outer surface that generally conforms to any suitable shape (for example, polygonal, conical, or others). A plurality of indents 106 can be disposed circumferentially on an outer wall 108 of the head portion 102. The shape and/or size of the head portion 102, and/or the plurality of indents 106, can facilitate a user's handling of the therapeutic delivery device 100 by the head portion 102. The outer wall 108 of the head portion 102 can be generally coaxial with a longitudinal axis of the therapeutic delivery device 100.

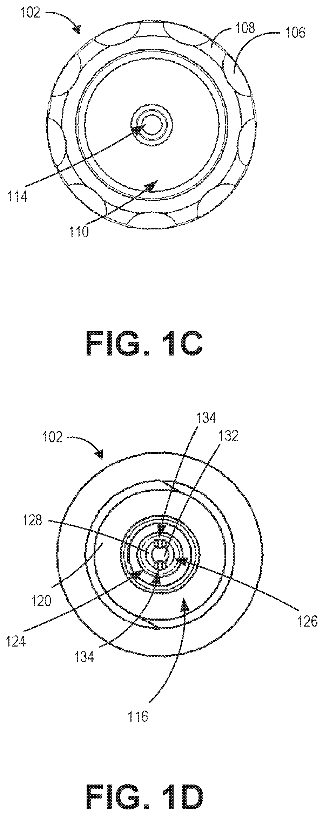

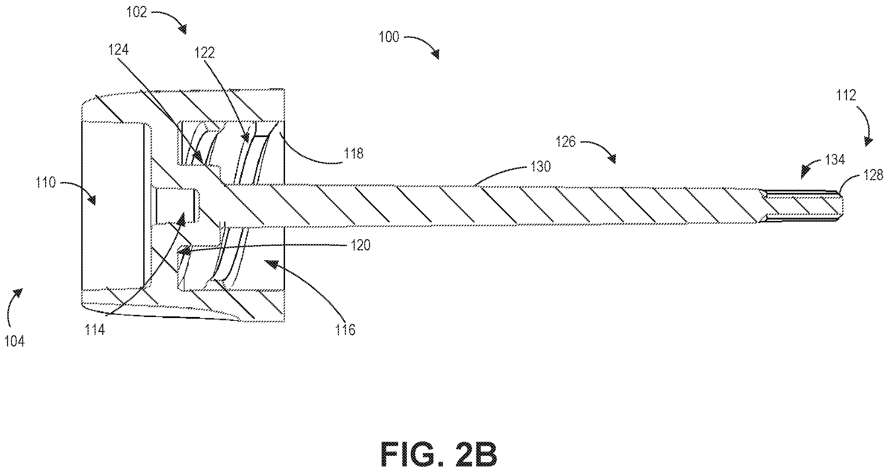

As shown in Figures 1A, 1C, 2A, and 2B, the head portion 102 can include a proximally facing recess 110 at the proximal end 104 of the therapeutic delivery device 100. The proximally facing recess 110 can have a first internal diameter and can be generally coaxial with the longitudinal axis of the therapeutic delivery device 100. The proximally facing recess 110 can extend from the proximal end 104 of the therapeutic delivery device 100 toward a distal end 112 of the therapeutic delivery device 100 and can terminate at a first depth. A second hole 114 can extend from the first depth to a second depth in the direction from the proximal end 104 toward the distal end 112 of the therapeutic delivery device 100. The second hole 114 can have an internal diameter that is smaller than the internal diameter of the proximally facing recess 110. The second hole 114 can be generally coaxial with the longitudinal axis of the therapeutic delivery device 100.

The sizes and/or shapes of the proximally facing recess 110 and the second hole 114 can be varied. In some embodiments, such as shown in Figures 2A and 2B, the proximally facing recess 110 and the second hole 114 are configured such that the head portion 102 can have a substantially uniform wall thickness. In some embodiments, the head portion 102 can be made substantially of plastic, which can have different shrinkage rates at different wall thicknesses. Having a substantially uniform wall thickness can promote even cooling and/or reduce warping of the head portion 102 during cooling. In some embodiments, the head portion 102 can have a general disc-shape or any other shape. In some embodiments, the proximally facing recess and/or second hole may be omitted while the head portion still has a substantially uniform wall thickness. In some embodiments, one or more additional structures or features can be positioned partially or entirely within the recess 110 and/or hole 114. For example, a negative-taste agent, such as a bitter agent (e.g., denatonium, denatonium benzoate such as Bitrex or Aversion, etc.) can be included in the recess 110 and/or hole 114, applied directly to the recess 110 and/or hole 114, or in a carrier positioned partially or entirely within the recess 110 and/or hole 114. The negative taste agent can be configured to discourage patients or others, especially young children, from swallowing or choking on the therapeutic delivery device 100 by inducing a gag reflex or expelling the device 100 if placed in the mouth. In some embodiments, another medical implement, such as a cap or a disinfecting swabbing pad can be temporarily or permanently positioned in a proximal region of the head portion 102, such as in the recess 110 and/or hole 114, to facilitate covering or sealing or disinfecting a medical connector 300 before or after use.

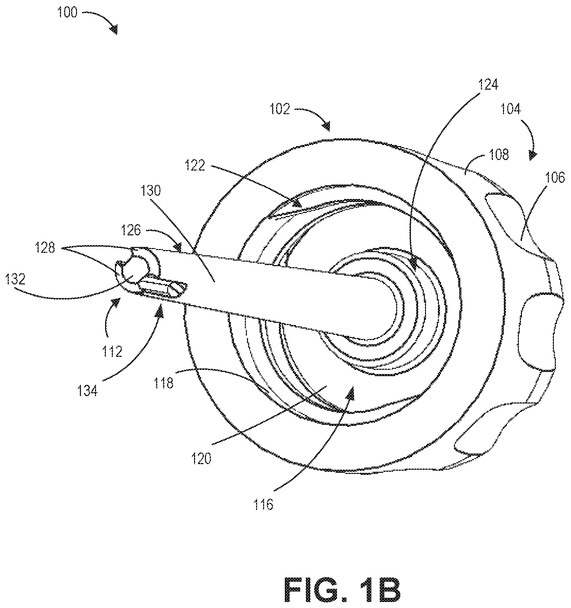

With reference to Figures 1B, 1D, 2A, and 2B, the head portion 102 can include a distally facing recess 116. The distally facing recess 116 can comprise an inner wall 118 and a distally facing end surface 120. The distally facing recess 116 can have an internal diameter that is approximately the same size as the proximally facing recess 110 such that the head portion 102 can have a substantially uniform wall thickness. In some embodiments, threads 122 can be formed on the inner wall 118. In some embodiments, the internal threads 122 can be configured to engage threads on a proximal end of a medical connector when the therapeutic delivery device 100 is applied to a medical connector. In some embodiments, the head portion 102 can have a general disc-shape or other flat shape. In some embodiments, the distally facing recess 116 and its associated features disclosed herein (such as the threads, the inner wall, or others) may be omitted.

A connecting base 124 can extend distally from the distally facing end surface 120. The connecting base 124 can be generally cylindrical, or have any other shape. The connecting base can have a substantially uniform cross-section along the longitudinal axis of the therapeutic delivery device 100. The connecting base 124 can have an outer diameter smaller than the internal diameter of the distally facing recess 116. The connecting base 124 can be generally coaxial with the longitudinal axis of the therapeutic delivery device 100. The connecting base 124 can have a length shorter than a depth of the distally facing recess 116. The depth of the distally facing recess 116 can be configured such that the head portion 102 can have a substantially uniform wall thickness. In some embodiments, such as shown in Figures 2A and 2B, the distally facing end surface 120 can be located between the first depth and the second depth such that the head portion 102 can have a substantially uniform wall thickness. In some embodiments, the connecting base 124 can interface with a valve member of a medical connector when the therapeutic delivery device 100 is attached to the medical connector. The outer diameter of the connecting base 124 can be large enough to allow a slit on the valve member to be pressed against a distal surface of the connecting base 124 along an entire length of the slit to form a seal, without opening the slit, without opening the slit enough to prevent a seal between the slit and the distal surface of the connecting base 124, and/or without permitting the connecting base 124 to enter into the slit.

With continued reference to Figures 1B, 1D, 2A, and 2B, an elongate shaft or projection 126 can extend distally from the distal surface of the connecting base 124. A distal end of the elongate shaft 126 can define the distal end 112 of the therapeutic delivery device 100. In some embodiments, the head portion 102, the connecting base 124, and the elongate shaft 126 can form a single piece.

The elongate shaft 126 can have an outer diameter that is configured to be slidably received in an internal fluid path of the medical connector, such as within the slit of the valve member. In some embodiments, the outer diameter of the elongate shaft 126 can be larger than the length of the slit of the valve member in its closed position along the proximal end of the valve member, such that the slit is forced to stretch to tightly receive the elongate shaft 126 (e.g., in a manner that is fluid-tight under pressures encountered during normal use). In some embodiments, such as shown in Figures 1B, 1D, 2A, and 2B, the elongate shaft 126 can have an outer diameter that is smaller than the outer diameter of the connecting base 124. The elongate shaft 126 can have a length such that when fully inserted into the medical connector, a distal portion of the elongate shaft 126 extends distally from a distal end of the medical connector. The elongate shaft 126 can have a substantially uniform cross-sectional width or diameter along a majority or substantially all or all of the longitudinal axis of the therapeutic delivery device 100 or, in some embodiments, such as shown in Figures 1B, 2A, and 2B, can have a taper (for example, a gradual taper) such that a proximal end (or base-connecting end) of the elongate shaft 126 is thicker than the distal end (or free end) of the elongate shaft 126. In some embodiments, such as shown in Figures 1B and 2A, the distal end of the elongate shaft 126 can include a chamfer 128. The chamfer 128 can have a steeper slope than the taper of the elongate shaft 126. The chamfer 128 and/or the taper along the length of the elongate shaft 126 can advantageously facilitate advancing the elongate shaft 126 through the valve member of the medical connector by pushing open the slit on the valve member, as will be described in greater detail below.

The elongate shaft 126 can have a shaft surface 130 along the longitudinal axis of the therapeutic delivery device 100 and a distal surface 132 at its distal end. At least a portion of the delivery device 100, such as the shaft surface 130 and/or the distal surface 132, can include one or more therapeutic agents (for example, an antimicrobial agent, an antibiotic, an antiseptic, an analgesic, an anesthetic, a blood-thinner, a chemotherapy drug, an immunosuppressive drug, a nutritional supplement, and/or any other therapeutic substance), such as in or on a coating on the shaft surface 130 or the distal surface 132, or temporarily or permanently embedded or impregnated on the shaft surface 130 or the distal surface 132.

In some embodiments, one or more therapeutic agents are provided on a portion, on a majority, and/or on substantially all of one or more surfaces or all of the therapeutic delivery device 100 (e.g., in a coating or in any other form of attachment), including on the elongate shaft 126, the connecting base 124, the distally facing surface 120, and/or the inner or outer walls 118, 108 of the head portion 102. When the catheter line is clamped, it is preferred that the clamp is applied after insertion of the therapeutic delivery device into the connector to avoid outside spilling of the liquid from within the connector or catheter. In some embodiments, such as if the clamp is applied before insertion of the therapeutic delivery device into the connector, the elongate shaft 126 can displace a volume of fluid when inserted into the medical connector. The displaced fluid can move toward and out of the proximal end of the connector, and flow on or near the top region of the medical connector (e.g., including a threaded portion in the top region of the medical connector), and/or over the inner wall 118 of the head portion 102 of the therapeutic delivery device 100. In some embodiments, the one or more therapeutic agents on the elongate shaft 126, the connecting base 124, the distally facing surface 120, and/or the inner wall 118 can be released into the displaced fluid and help to disinfect the top surface, the top region, and/or the thread area in a proximal connecting region of the medical connector.

In some embodiments, the one or more therapeutic agents can include antimicrobial materials (for example, chlorhexidine, chlorhexidine gluconate (CHG), vancomycin, cefazolin, ceftazidime, ciprofloxacin, gentamicin, ampicillin, one or more metal ions (e.g., silver and/or copper ions), and/or one or more other agents with antimicrobial properties).

In some embodiments, the one or more therapeutic agents can include one or more antimicrobial materials including but not limited to metal materials such as silver or copper ions (for example, silver or copper nanoparticles, ionic silver or copper, or otherwise) embedded within, formed as part of, or compounded with a plastic, resin, polymer, and/or elastomeric base material. Antimicrobial materials can be embedded in, formed as part of, or compounded with at least a portion or substantially all of the therapeutic delivery device 100 (such as at least a portion of the elongate shaft 126), which can be made of the plastic, resin, polymer, and/or elastomeric base material. The one or more antimicrobial materials can be infused or added into the base material during molding or formation of the base material. In some embodiments, the one or more antimicrobial materials are evenly or substantially evenly distributed throughout the base material. In some embodiments, the one or more antimicrobial materials are embedded in, formed as part of, or compounded with the base material without being necessarily bonded to the base material. In some embodiments, the one or more therapeutic agents can elute from the antimicrobial base material when exposed to a fluid (such as saline). Alternatively and/or additionally, the embedded or impregnated therapeutic agent may not be released into the fluid. In some embodiments, the base material can include one or more of any other suitable antimicrobial materials, such as any of those disclosed herein. In some embodiments, the elongate shaft of the therapeutic delivery device can be made entirely of metal, such as copper, silver, or alloys including copper and/or silver.

In some embodiments, the one or more therapeutic agents can be coated on the one or more surfaces or all of the therapeutic delivery device 100. In some embodiments, the coated therapeutic agents can come into contact with a fluid (for example, saline, heparin, water, blood, or others) in a catheter coupled to the medical connector when the therapeutic delivery device 100 is applied to the medical connector. The one or more therapeutic agents can be released into the fluid in the connector and/or catheter to form a locking solution (such as an antimicrobial locking solution). In some embodiments, the elongate shaft 126 can be configured to include a coating or other structure or composition with a therapeutic agent concentration of at least about 15% or at least about 20% (by weight, mole, or volume), which can be configured to provide, when dissolved or leached out or otherwise released, a locking solution with a concentration of therapeutic agent in the connector and/or catheter of less than or equal to about 3%, or less than or equal to about 2%, or less than or equal to about 1% (by weight, mole, or volume). In some embodiments, the concentration of therapeutic agent can be at least about 0.5 mg/mL, 1.0 mg/mL, 2.5 mg/mL, 5.0 mg/mL, 10 mg/mL, 15 mg/mL, 40 mg/ML, values between the aforementioned values, ranges spanning those values, or otherwise. In some embodiments, the elongate shaft 126 can be provided with at least about: 0.2 mg, 0.5 mg, 1.0 mg, 2.0 mg, 2.5 mg, 5.0 mg, 7.5 mg, or 20 mg of one or more therapeutic agents, values between the aforementioned values, ranges spanning those values, or otherwise.

Attaching the one or more therapeutic agents to the therapeutic delivery device 100 can be performed by any suitable method. For example, in some embodiments, the one or more therapeutic agents can be impregnated or dispersed or coated in or over at least a portion of the shaft surface 130 and/or the distal surface 132. In some embodiments, the attachment of the one or more therapeutic agents can be accomplished by dipping at least a portion of the elongate shaft 126 (and/or the proximal end 102 and/or the threads 122, etc.) into a therapeutic agent solution, or by spraying one or more therapeutic agents onto the elongate shaft 126 and/or other portions of the device, or by including one or more therapeutic agents in the constituents forming the elongate shaft 126 and/or other portions of the device, and/or binding the one or more therapeutic agents through one or more binders to the elongate shaft 126 and/or other portions of the device. After attachment of the one or more therapeutic agents, the elongate shaft 126 can be dried, such as in part of a dip-coat or other process. The drying step can be accomplished by simple air drying in a room temperature range, or by heated drying, or by "freeze drying" or lyophilization, etc. In some embodiments, the weight of the attached therapeutic agent can be controlled by using a precision balance, comparing the weight of the therapeutic delivery device 100 before application of the therapeutic agent with the weight of the therapeutic delivery device 100 during or after application of the therapeutic agent.

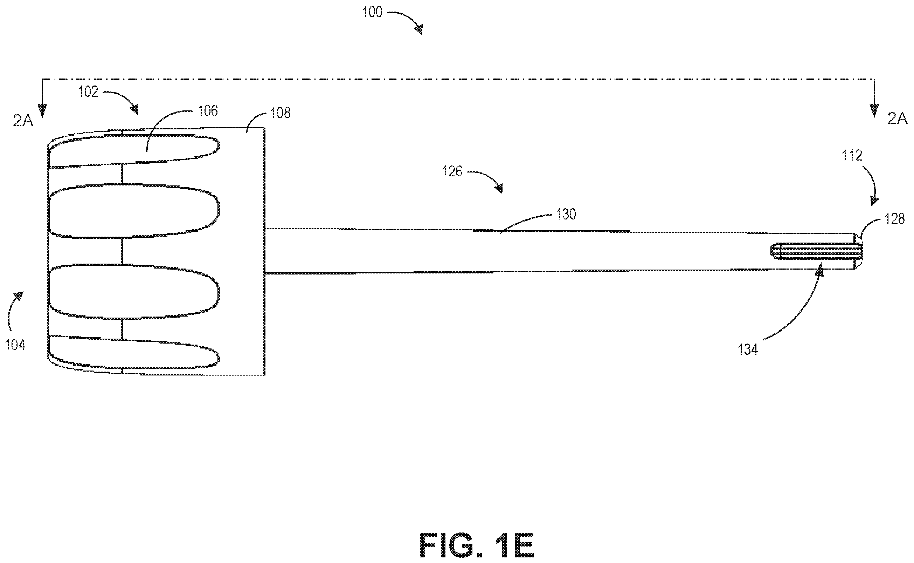

In some embodiments, at least the portion(s) of the surfaces of the elongate shaft 126 that is(are) coated with the one or more therapeutic agents can include one or more uneven or non-smooth surfaces (for example, roughened, textured, knurled, perforated, indented, pitted, having one or more ridges, grooves, slits, troughs, and/or protrusions, or otherwise). In some embodiments, at least the portion(s) of the surfaces of the elongate shaft 126 that is(are) coated with the one or more therapeutic agents can include a hollow interior. The surface coated with the one or more therapeutic agents can be increased by the one or more uneven or non-smooth surfaces, and/or the hollow interior. The recesses and/or hollow interior can provide an aggregate volume sufficient to retain an amount of therapeutic agent(s) that is clinically effective (for example, against CRBSI). In some embodiments, such as shown in Figures 1A, 1B, 1D, 1E, and 2A-2B, a plurality of (for example, two, four, or others) grooves or flutes 134 can extend along at least a portion of the elongate shaft 126, such as from the distal surface 132 toward the proximal end 102 of the therapeutic delivery device 100. As shown, the plurality of grooves or flutes 134 can be located substantially symmetrically about a circumference of the elongate shaft 126. In some embodiments, the plurality of groove or flutes 134 can extend more proximally than as shown in the figures. For example, the plurality of grooves or flutes 134 can extend along a majority of the length of the elongate shaft 126 or substantially along the entire length of the elongate shaft 126. In some embodiments, the grooves or flutes can be helical or otherwise. The uneven or non-smooth surfaces can have other shapes and/or sizes. The uneven or non-smooth surfaces can provide a region in which one or more therapeutic agents can be retained during insertion of the elongate shaft 126 into the seal of the connector, reducing the likelihood that one or more attached therapeutic agents will be substantially wiped off of or otherwise removed from the elongate shaft 126 as the elongate shaft 126 advances through the valve member of the medical connector.

### Example Assemblies of Therapeutic Device and Medical Connector

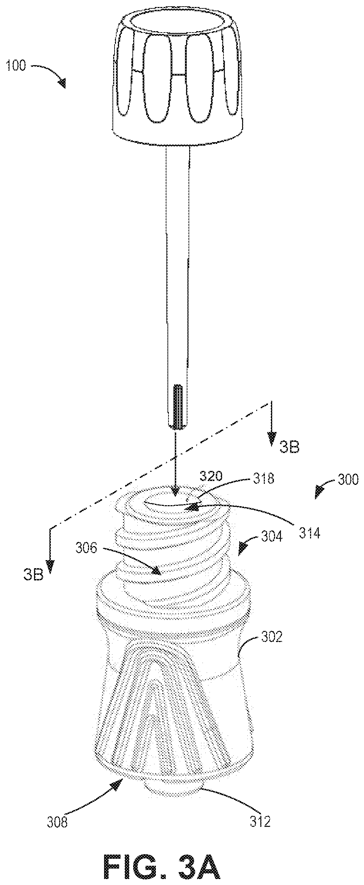

Figures 3A to 3E illustrate an example of applying the therapeutic delivery device 100 described above to an example medical connector 300. Although not all aspects of the therapeutic delivery device 100 are labeled, it is understood that unless described otherwise, features illustrated as in previous embodiments will be structured and will operate in a manner that is the same as or substantially similar to those previously described.

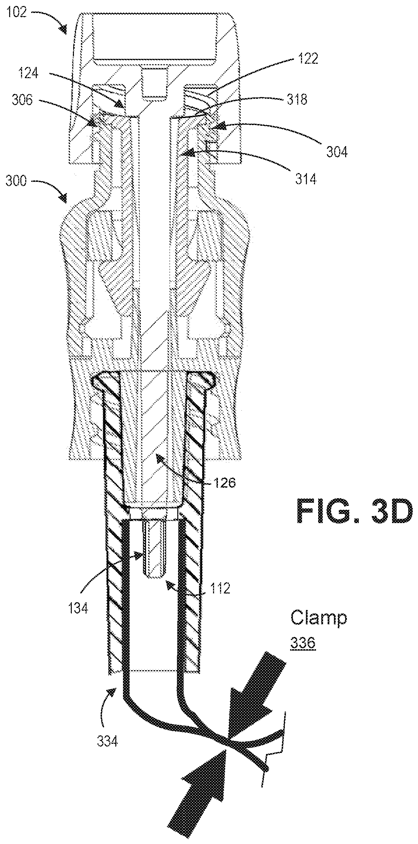

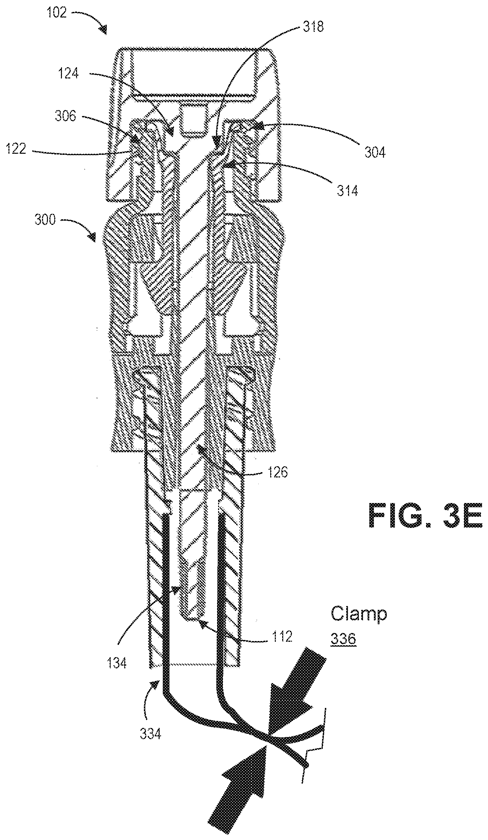

The medical connector 300 can include an outer housing 302. The outer housing 302 can be substantially rigid. The housing 302 can have a proximal Luer connector region 304 with threads 306 for receiving a threaded medical connector (such as a Luer connector) of a medical device (such as a syringe) when the medical connector is used in a fluid pathway. The housing 302 can have a distal Luer connector region 308, which can include threads 310 and a Luer cannula 312. In some embodiments, such as shown in Figures 3D and 3E, the distal Luer connector region 308 can be coupled (such as releasably coupled) to a catheter 334 or catheter assembly (for example, a hemodialysis catheter or catheter assembly). In various embodiments, the proximal and distal connector regions 304, 308 can generally be configured to accommodate any standard medical connector or implement, and can be configured to conform with ISO or ANSI or other applicable standards. The term "medical implement" is used herein to denote any medical device used in the medical field that can be connected or joined with any embodiments of the connectors disclosed herein.

In some embodiments, fluid flowing through a medical connector at high flow rates (for example, at least about 450 milliliters per minute) can develop air bubbles, especially when flowing from a lower position to a higher position (such from the distal Luer connector region 308 to the proximal Luer connector region 304 when the medical connector 300 is in an upright position) and/or when there is a sudden change in a cross-sectional area of the fluid pathway. The air bubbles can increase hemolysis of a patient's blood flowing through the medical connector. The Luer cannula 312 of the medical connector 300 can have a generally, substantially, or entirely straight, and/or non-tortuous fluid pathway having an internal diameter that is substantially the same as or similar to an internal diameter of an outflow port of a medical implement coupled to the medical connector 300 at the proximal Luer connector region 304. The straight fluid pathway can reduce turbulence in the blood flow, which can reduce the development of such bubbles and/or reduce a rate of hemolysis. Additional details of the medical connector 300 are described in U.S. Patent Application No. 14/708,098, filed May 8, 2015, published as U.S. Patent Application Publication No. 2016/0001056.

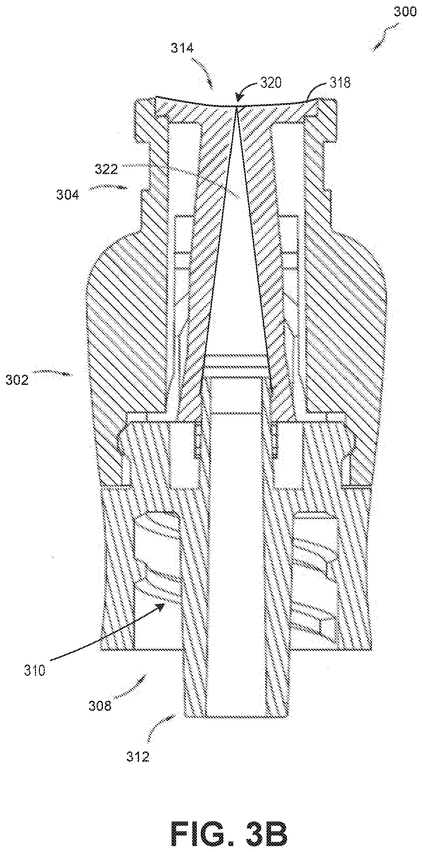

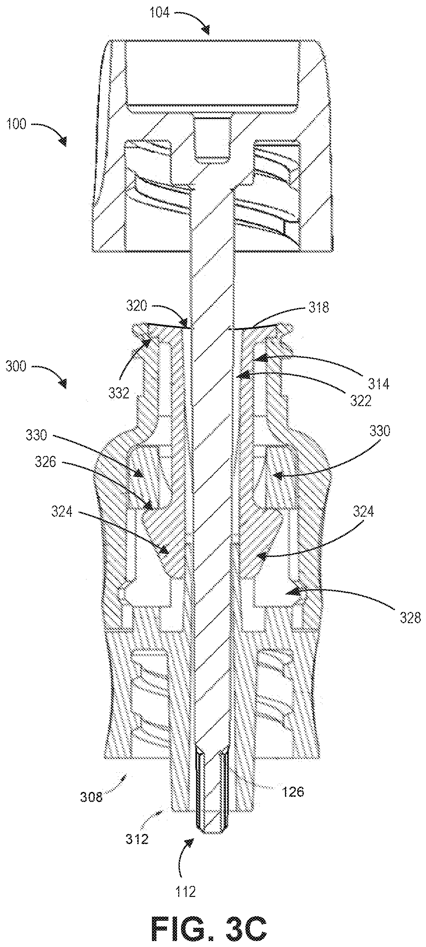

The medical connector 300 can include a seal or valve member 314 configured to be positioned at least partially within the outer housing 302. The seal or valve member 314 can have a top or proximal end 318 with a normally closed slit 320 that extends through the top or proximal end 318 and into a cavity 322 (see Figures 3B and 3C) within the valve member 314. The slit 320 can comprise a region within the seal or valve member 314 that, when the seal or valve member 314 is closed, begins at the proximal end of the seal or valve member 314 and extends distally within the seal or valve member 314. As shown, the region within the slit 320 is not exposed to the region outside of the connector 300 when the seal or valve member 314 is closed. The valve member 314 can be configured to receive the therapeutic delivery device 100. As shown in the cross-section of a fully assembled medical connector 300 along a plane perpendicular to the slit 320 in Figure 3B, the slit 320 can form a seal against entry into the cavity 322 when the medical connector 300 is in a closed position. The slit 320, when in an open position, can be in fluidic communication with the Luer cannula 312. As shown in the cross-section of a fully assembled medical connector 300 along the slit 320 in Figure 3C, the valve member 314 can be stretched downward toward the Luer cannula 312 such that shoulders 324 of the valve member 314 can move down and a top surface 326 of the shoulders 324 can reach openings 328. The top surface 326 of the shoulders 324 can be pressed proximally against the surface of upward projections 330, while the top or proximal end 318 of the valve member 314 can be pressed distally against ledges 332 of the housing 302. Accordingly, the valve member 314 can be tensioned along a longitudinal axis of the medical connector 300, which can create compression in a plane perpendicular to the longitudinal axis at the top end 318 of the valve member 314. The tension can make the sides of the slit 320 press more tightly together than when the valve member 314 is not stretched downward as described in U.S. Patent Application Publication No. 2016/0001056, increasing the amount of fluid pressure that the slit 3200 can resist during the process of inserting a catheter into a patient and after a medical implement (such as a syringe) is removed.

With continued reference to Figure 3C, as the distal end 112 of the therapeutic delivery device 100 reaches the slit 320, the distal end 112 of the therapeutic delivery device 100 can push through the slit 320 and enter the cavity 322. As described above, the chamfer 128 on the distal end of the elongate shaft 126 can make it easier to push aside the two sides of the slit 320. The elongate shaft 126 of the therapeutic delivery device 100 can extend past the valve member 314, into the straight lumen of the Luer cannula 312, and distally from a distal end of the Luer cannula 312.

Figures 3D and 3E illustrate the therapeutic delivery device 100 at least substantially fully inserted into the medical connector 300. As shown in Figures 3D and 3E, at the proximal end of the medical connector 300, the distal surface of the connecting base 124 of the therapeutic delivery device 100 can abut and/or be pressed into contact with the top or proximal end 318 of the valve member 314. As shown in Figure 3E, a portion of the top or proximal end 318 of the valve member 314 can be stretched downward into contacting and/or partially or completely surrounding a side surface of the connecting base 124, while the connecting base 124 still remains outside of the slit 320. The outer diameter of the connecting base 124 can be substantially greater than a length of the slit 320 such that the connecting base 124 does not enter the slit 320 in normal use. A seal can be formed by the contact between the top or proximal end 318 of the valve member 314 (when downwardly stretched or not) and the distal surface of the connecting base 124 (and/or the side surface of the connecting base 124). In some embodiments, as shown, the connecting base 124 does not: (a) enter into the region within the slit or the interior of the slit of the seal or valve member; (b) form a seal with the region within the slit or the interior of the slit of the seal or valve member; (c) form a seal with a female Luer-receiving surface; and/or (d) form a seal with and/or contact any rigid portion of the connector during normal use.

More specifically, the top or proximal end 318 of the valve member 314, which is made of a more deformable and/or resilient material than the connecting base 124, can conform around the distal surface and/or the side surface of the connecting base 124 to form the seal. In the illustrated embodiment, the connecting base 124 does not form a seal within the cavity 322 of the valve member 314. The threads 122 on the head portion 102 of the therapeutic delivery device 100 can at least partially engage the threads 306 on the proximal Luer connector region 304 of the medical connector 300. The engagement between the threads 122 and the threads 306 can keep the therapeutic delivery device 100 coupled to the medical connector 300 and/or maintain the seal between the valve member 314 and the distal surface of the connecting base 124.

At the distal end of the medical connector 300, at least a distal portion of the elongate shaft 126, and/or at least portions of the plurality of groove or flute 134 can extend distally from the distal end of the Luer cannula 312. In some embodiments, the distal Luer connector region 308 can be coupled to a catheter, such as the catheter 334 in Figures 3D and 3E, or a catheter system. The therapeutic agents on or in at least portions of the elongate shaft 126 can come into contact with a fluid inside the catheter 334. The therapeutic agents can be released into the fluid to form a locking solution. In some embodiments, the therapeutic agents can be released slowly over time from the surface(s) of the elongate shaft 126 at a predetermined release rate so as to provide increasing or generally stable locking concentration (such as increasing or generally stable antimicrobial locking concentration) substantially throughout the duration between uses of the catheter for delivering fluid to and from the patient's blood vessel.

In some embodiments, such as shown in Figures 3D and 3E, a tubing clamp 336, or any other mechanisms for stopping fluid flow in a catheter, can be applied to the catheter 334 at a location proximal to the medical connector 300 and to the distal end 112 of the therapeutic delivery device 100. In some embodiments, the clamp 336 can be applied after the therapeutic delivery device 100 has been substantially and/or fully inserted into the medical connector 300. Otherwise, if the clamp were applied before insertion of the therapeutic delivery device 100, liquid could leak or be expelled out of the proximal end of the medical connector 300, since the liquid displaced by the insertion of the therapeutic delivery device 100 would otherwise not be able to migrate distally past the clamp and would therefore migrate proximally. The application of the clamp 336 can result in at least about 0.2 mL, 0.5 mL, 0.8 mL, or other values of a locking solution being substantially retained at the proximal end of the catheter 334.

An example of a method of performing a lock procedure in a catheter configured to be coupled to a patient's body for an extended period of time (for example, for 2-3 days to about a week, or for any other suitable amount of time) will now be described. A distal end of the catheter is located within a patient (such as being implanted in a blood vessel) and a proximal end of the catheter can be located outside of the patient. The proximal end of the catheter (for example, a female Luer connector at the proximal end of the catheter) can be coupled to a distal end of a medical connector described herein, such as the medical connector 300 of Figures 3A-3E. A user can inject a volume of a fluid (for example, saline, heparin, or others) into a lumen of the catheter via connection between a medical implement (such as a syringe) and the medical connector.

After injecting the fluid into the catheter, the user can withdraw the medical implement from the medical connector and insert a therapeutic delivery device, such as the therapeutic delivery device 100 described herein, into the medical connector. The distal end of the elongate shaft of the therapeutic delivery device can be configured to open a slit on the valve member of the medical connector to enter a cavity in the valve member. The user can continue to advance the therapeutic delivery device distally into a Luer catheter of the medical connector. When substantially fully inserted, the distal end of the elongate shaft extends distally from the distal end of the medical connector and a head portion of the medical connector engages the proximal end of the medical connector. The user can rotate the therapeutic delivery device about its longitudinal axis to threadedly engage threads on the head portion of the therapeutic delivery device with threads on the proximal Luer connector region of the medical connector. In some embodiments, such as shown in Figure 3D, the user can rotate the head portion slightly more (for example, by another quarter turn) when the distal surface of the connecting base comes into contact with the top or proximal end of the valve member to establish a seal. In some embodiments, the user can fully engage the threads on the head portion of the therapeutic delivery device and the proximal connecting region of the medical connector until the proximal end of the medical connector contacts or is almost contacting the distally facing surface of the head portion. A base member of the therapeutic delivery device can be pressed into contact with the valve member of the medical connector to form a seal against the cavity in the valve member. One or more therapeutic agents (such as antimicrobial materials) coated on at least portions of the elongate shaft of the therapeutic delivery device can come into contact with and be released into the injected fluid to form a locking solution.

Once the therapeutic delivery device is partially, substantially, and/or fully inserted into the medical connector, the user can apply a clamp across a portion of the catheter outside the patient. The clamp can be applied to the catheter at a location distal of the medical connector and the distal end of the therapeutic delivery device. The clamp can substantially prevent fluidic communication between portions of the catheter proximal and distal of the clamp.

In some embodiments, the medical connector 300 can include one or more therapeutic agents. In some embodiments, any suitable antimicrobial material, such as any of the antimicrobial materials disclosed herein, can be coated or otherwise included on a surface of the internal fluid path of the medical connector 300. In some embodiments, the valve member 314 can include an antimicrobial flexible or resilient valve base material (such as silicone or otherwise). In some embodiments, the valve material can include a metal material, such as silver or copper (for example, silver or copper nanoparticles, ionic silver or copper, or otherwise) embedded within, formed as part of, or compounded with the valve material. In some embodiments, the Luer cannula 312 can include an antimicrobial plastic, polymer, elastomer, or resin base material with antimicrobial material, such as metal material embedded within, formed as part of, or compounded with the base material. In some embodiments, both the valve member 314 and the Luer cannula 312 can include antimicrobial material included with the valve material and/or the plastic or resin base material respectively. The antimicrobial material can be infused or otherwise added during manufacturing of the valve material and/or molding or formation of the plastic or resin. In some embodiments, the antimicrobial material evenly or substantially evenly distributed throughout the valve material and/or the base material. In some embodiments, the valve material and/or the base material can be compounded or included with any other suitable antimicrobial material, including one or more of those disclosed herein.

In some embodiments, the antimicrobial materials are embedded, compounded, or otherwise added without being necessarily bonded to the valve material or the base material. The antimicrobial valve member and/or the antimicrobial Luer cannula can elute the antimicrobial materials when a fluid (such as saline) flows through the internal fluid path in the medical connector 300. Alternatively and/or additionally, one or more of the antimicrobial materials may not be released into the fluid.

In some embodiments, the combination of the antimicrobial materials disposed on the therapeutic delivery device 100 and in the medical connector 300 can provide a sufficient concentration of antimicrobial materials to form an antimicrobial lock. In some embodiments, any one of the therapeutic agents disposed on the therapeutic delivery device 100 or in the medical connector 300 can each provide sufficient antimicrobial materials to form an antimicrobial lock. The therapeutic delivery device 100 can be assembled with the medical connector 300 that is coated and/or embedded with antimicrobial materials to provide redundancy.

As shown, in some embodiments, the structural portions of the therapeutic delivery device 100 comprise an integrally formed unitary component, made of a single piece of material. As illustrated, the therapeutic delivery device 100 does not include any mechanically moving parts or liquid reservoirs or mixing receptacles. In the illustrated example, the therapeutic delivery device 100 is configured to be inserted or removed from the connector 300 at any time by a user and is not a permanent part of the connector 300.

Certain features that are described in this disclosure in the context of separate implementations can also be implemented in combination in a single implementation. Conversely, various features that are described in the context of a single implementation also can be implemented in multiple implementations separately or in any suitable subcombination. Moreover, although features may be described above as acting in certain combinations, one or more features from a claimed combination can in some cases be excised from the combination, and the combination may be claimed as a subcombination or variation of a subcombination.

Any portion of any of the steps, processes, structures, and/or devices disclosed or illustrated in one embodiment, flowchart, or example in this disclosure can be combined or used with (or instead of) any other portion of any of the steps, processes, structures, and/or devices disclosed or illustrated in a different embodiment, flowchart, or example. The embodiments and examples described herein are not intended to be discrete and separate from each other. Combinations, variations, and other implementations of the disclosed features are within the scope of this disclosure.

Some embodiments have been described in connection with the accompanying drawings. Moreover, while operations may be depicted in the drawings or described in the specification in a particular order, such operations need not be performed in the particular order shown or in sequential order, and/or one or more of the operations may be omitted entirely, to achieve desirable results. Other operations that are not depicted or described can be incorporated in the example methods and processes. For example, one or more additional operations can be performed before, after, simultaneously, or between any of the described operations. Additionally, the operations may be rearranged or reordered in other implementations. Also, the separation of various components in the implementations described above should not be understood as requiring such separation in all implementations, and it should be understood that the described components and systems can generally be integrated together in a single product or packaged into multiple products. Additionally, other implementations are within the scope of this disclosure.

For purposes of this disclosure, certain aspects, advantages, and novel features are described herein. Not necessarily all such advantages may be achieved in accordance with any particular embodiment. Thus, for example, those skilled in the art will recognize that the disclosure may be embodied or carried out in a manner that achieves one advantage or a group of advantages as taught herein without necessarily achieving other advantages as may be taught or suggested herein.

Conditional language, such as "can," "could," "might," or "may," unless specifically stated otherwise, or otherwise understood within the context as used, is generally intended to convey that certain embodiments include, while other embodiments do not include, certain features, elements, and/or steps. Thus, such conditional language is not generally intended to imply that features, elements, and/or steps are in any way required for one or more embodiments or that one or more embodiments necessarily include logic for deciding, with or without user input or prompting, whether these features, elements, and/or steps are included or are to be performed in any particular embodiment.

Conjunctive language such as the phrase "at least one of X, Y, and Z," unless specifically stated otherwise, is otherwise understood with the context as used in general to convey that an item, term, etc. may be either X, Y, or Z. Thus, such conjunctive language is not generally intended to imply that certain embodiments require the presence of at least one of X, at least one of Y, and at least one of Z.

Language of degree used herein, such as the terms "approximately," "about," "generally," and "substantially" as used herein represent a value, amount, or characteristic close to the stated value, amount, or characteristic that still performs a desired function or achieves a desired result.

The scope of the present disclosure is not intended to be limited by the specific disclosures of preferred embodiments in this section or elsewhere in this specification. The invention is defined by the independent claims, to which attention is directed.

## Claims

1. A therapeutic delivery system for use in performing an antimicrobial lock procedure, the system comprising:
a medical fluid connector (300) having a proximal end and a distal end;
a selectively resealable valve member (314) comprising a normally closed slit (320), the valve member (314) configured to seal a proximal end of a catheter (334), the selectively resealable valve member (314) being located in the medical fluid connector (300) having the proximal end and the distal end; and
a therapeutic delivery device (100) including an elongate shaft (126) and a base portion (124), the elongate shaft (126) extending distally of the base portion, an outer diameter of the base portion (124) being greater than a length of the slit (320), one or more antimicrobial materials disposed on at least the elongate shaft (126),
wherein the elongate shaft (126) is configured to be inserted through the slit (320), the one or more antimicrobial materials disposed at least partially on an outer shaft surface (130) of the shaft (126) inserted through the slit (320), and
wherein, when assembled, contact between the base portion (124) and the valve member (314) at the slit (320) forms a seal, the one or more antimicrobial materials configured to be in contact with and released into a fluid in the catheter (334) to form an antimicrobial locking solution.

2. The system of Claim 1, wherein the medical fluid connector (300) comprises a non-tortuous fluid path.

3. The system of Claim 1 or 2, wherein the medical fluid connector (300) comprises a fluid path having an internal diameter that is substantially the same as an outlet port of a medical implement used to inject the fluid into the catheter (334).

4. The system of any of Claims 1-3, wherein the valve member (314) is located at or near a proximal end of the medical fluid connector (300).

5. The system of any of Claims 1-4, wherein the therapeutic delivery device (100) comprises a head portion (102) configured to engage a proximal end of the medical fluid connector (300) so as to press the base portion (124) into contact with the valve member (314) at the slit (320) to form the seal.

6. The system of any of Claims 1-5, wherein the medical fluid connector (300) is spike-less.

7. The system of any of Claims 1-6, wherein the base portion (124) is configured to be positioned proximal and outside of the slit (320) when the therapeutic delivery device (100) is fully attached to the medical fluid connector (300).

8. The system of any of Claims 1-7, wherein the medical fluid connector (300) is a needle-less connector.

9. The system of Claim 1, wherein a distal end of the elongate shaft (126) is configured to open the slit (320).

10. The system of any of Claims 1-9, wherein at least a portion of the elongate shaft (126) with the one or more antimicrobial materials disposed thereon comprises an uneven surface and/or one or more cavities.

11. The system of any of Claims 1-10, wherein the one or more antimicrobial materials are disposed on substantially an entire surface of the therapeutic delivery device (100).

12. The system of any of Claims 1-11, wherein the valve member (314) comprises a more deformable and/or more resilient material than the base portion (124) such that the valve member (314) is configured to conform around a distal and/or side surface (120) of the base portion (124) to form the seal.

13. The system of any of Claims 1-12, wherein the base portion (124) is cylindrical.

14. The system of any of Claims 1-13, wherein the base portion (124) has a substantially uniform cross-section along a longitudinal axis of the therapeutic delivery device (100).

## Patentansprüche

1. Therapeutisches Abgabesystem zur Verwendung bei der Durchführung eines antimikrobiellen Lock-Verfahrens, wobei das System Folgendes umfasst:
einen medizinischen Fluidverbinder (300) mit einem proximalen Ende und einem distalen Ende;
ein selektiv wiederverschließbares Ventilelement (314), umfassend einen normalerweise geschlossenen Schlitz (320), wobei das Ventilelement (314) dazu ausgebildet ist, ein proximales Ende eines Katheters (334) abzudichten, wobei sich das selektiv wiederverschließbare Ventilelement (314) in dem medizinischen Fluidverbinder (300) mit dem proximalen Ende und dem distalen Ende befindet; und
eine therapeutische Abgabevorrichtung (100), die einen länglichen Schaft (126) und einen Basisabschnitt (124) einschließt, wobei sich der längliche Schaft (126) distal von dem Basisabschnitt erstreckt, wobei ein Außendurchmesser des Basisabschnitts (124) größer als eine Länge des Schlitzes (320) ist, wobei ein oder mehrere antimikrobielle Materialien zumindest auf dem länglichen Schaft (126) angeordnet sind,
wobei der längliche Schaft (126) dazu ausgebildet ist, durch den Schlitz (320) eingeführt zu werden, wobei das eine oder die mehreren antimikrobiellen Materialien zumindest teilweise auf einer äußeren Schaftoberfläche (130) des durch den Schlitz (320) eingeführten Schafts (126) angeordnet sind, und
wobei im zusammengebauten Zustand ein Kontakt zwischen dem Basisabschnitt (124) und dem Ventilelement (314) an dem Schlitz (320) eine Abdichtung bildet, wobei das eine oder die mehreren antimikrobiellen Materialien dazu ausgebildet sind, mit einem Fluid in dem Katheter (334) in Kontakt zu stehen und in dieses freigesetzt zu werden, um eine antimikrobielle Lock-Lösung zu bilden.

2. System nach Anspruch 1, wobei der medizinische Fluidverbinder (300) einen nicht gewundenen Fluidpfad umfasst.

3. System nach Anspruch 1 oder 2, wobei der medizinische Fluidverbinder (300) einen Fluidpfad mit einem Innendurchmesser umfasst, der im Wesentlichen dem einer Auslassöffnung eines medizinischen Instruments entspricht, das zum Injizieren des Fluids in den Katheter (334) verwendet wird.

4. System nach einem der Ansprüche 1-3, wobei sich das Ventilelement (314) an oder nahe einem proximalen Ende des medizinischen Fluidverbinders (300) befindet.

5. System nach einem der Ansprüche 1-4, wobei die therapeutische Abgabevorrichtung (100) einen Kopfabschnitt (102) umfasst, der dazu ausgebildet ist, mit einem proximalen Ende des medizinischen Fluidverbinders (300) in Eingriff zu kommen, sodass der Basisabschnitt (124) an dem Schlitz (320) in Kontakt mit dem Ventilelement (314) gedrückt wird, um die Abdichtung zu bilden.

6. System nach einem der Ansprüche 1-5, wobei der medizinische Fluidverbinder (300) dornlos ist.

7. System nach einem der Ansprüche 1-6, wobei der Basisabschnitt (124) dazu ausgebildet ist, proximal und außerhalb des Schlitzes (320) positioniert zu sein, wenn die therapeutische Abgabevorrichtung (100) vollständig an dem medizinischen Fluidverbinder (300) angebracht ist.

8. System nach einem der Ansprüche 1-7, wobei der medizinische Fluidverbinder (300) ein nadelloser Verbinder ist.

9. System nach Anspruch 1, wobei ein distales Ende des länglichen Schafts (126) dazu ausgebildet ist, den Schlitz (320) zu öffnen.

10. System nach einem der Ansprüche 1-9, wobei zumindest ein Abschnitt des länglichen Schafts (126), auf dem das eine oder die mehreren antimikrobiellen Materialien angeordnet sind, eine unebene Oberfläche und/oder einen oder mehrere Hohlräume umfasst.

11. System nach einem der Ansprüche 1-10, wobei das eine oder die mehreren antimikrobiellen Materialien auf im Wesentlichen der gesamten Oberfläche der therapeutischen Abgabevorrichtung (100) angeordnet sind.

12. System nach einem der Ansprüche 1-11, wobei das Ventilelement (314) ein verformbareres und/oder elastischeres Material als der Basisabschnitt (124) umfasst, sodass das Ventilelement (314) dazu ausgebildet ist, sich um eine distale und/oder seitliche Oberfläche (120) des Basisabschnitts (124) anzuschmiegen, um die Abdichtung zu bilden.

13. System nach einem der Ansprüche 1-12, wobei der Basisabschnitt (124) zylindrisch ist.

14. System nach einem der Ansprüche 1-13, wobei der Basisabschnitt (124) entlang einer Längsachse der therapeutischen Abgabevorrichtung (100) einen im Wesentlichen gleichmäßigen Querschnitt aufweist.

## Revendications

1. Système d'administration thérapeutique destiné à être utilisé dans la réalisation d'une procédure de blocage antimicrobien, le système comprenant :
un raccord (300) de fluide médical présentant une extrémité proximale et une extrémité distale ;
un organe (314) de valve rescellable sélectivement comprenant une fente (320) normalement fermée, l'organe (314) de valve étant configuré pour sceller une extrémité proximale d'un cathéter (334), l'organe (314) de valve rescellable sélectivement situé dans le raccord (300) de fluide médical présentant l'extrémité proximale et l'extrémité distale ; et
un dispositif d'administration thérapeutique (100) incluant un arbre (126) allongé et une portion de base (124), l'arbre (126) allongé s'étendant distalement de la portion de base, un diamètre externe de la portion de base (124) étant supérieur à une longueur de la fente (320), un ou plusieurs matériaux antimicrobiens étant disposés sur au moins l'arbre (126) allongé,
dans lequel l'arbre (126) allongé est configuré pour être inséré à travers la fente (320), les un ou plusieurs matériaux antimicrobiens étant disposés au moins partiellement sur une surface externe (130) d'arbre de l'arbre (126) inséré à travers la fente (320), et
dans lequel, une fois assemblés, le contact entre la portion de base (124) et l'organe (314) de valve au niveau de la fente (320) forme une étanchéité, les un ou plusieurs matériaux antimicrobiens étant configurés pour être en contact avec un fluide dans le cathéter (334) et s'y libérer afin de former une solution de blocage antimicrobien.

2. Système selon la revendication 1, dans lequel le raccord (300) de fluide médical comprend un trajet de fluide non tortueux.

3. Système selon la revendication 1 ou la revendication 2, dans lequel le raccord (300) de fluide médical comprend un trajet de fluide présentant un diamètre interne qui est sensiblement identique à un orifice de sortie d'un outil médical utilisé pour injecter le fluide dans le cathéter (334).

4. Système selon l'une quelconque des revendications 1 à 3, dans lequel l'organe (314) de valve est situé au niveau ou près d'une extrémité proximale du raccord (300) de fluide médical.

5. Système selon l'une quelconque des revendications 1 à 4, dans lequel le dispositif d'administration thérapeutique (100) comprend une portion de tête (102) configurée pour venir en prise avec une extrémité proximale du raccord (300) de fluide médical de manière à presser la portion de base (124) en contact avec l'organe (314) de valve au niveau de la fente (320) pour former l'étanchéité.

6. Système selon l'une quelconque des revendications 1 à 5, dans lequel le raccord (300) de fluide médical est sans pointe.

7. Système selon l'une quelconque des revendications 1 à 6, dans lequel la portion de base (124) est configurée pour être positionnée proximale et à l'extérieur de la fente (320) lorsque le dispositif d'administration thérapeutique (100) est entièrement fixé au raccord (300) de fluide médical.

8. Système selon l'une quelconque des revendications 1 à 7, dans lequel le raccord (300) de fluide médical est un raccord sans aiguille.

9. Système selon la revendication 1, dans lequel une extrémité distale de l'arbre (126) allongé est configurée pour ouvrir la fente (320).

10. Système selon l'une quelconque des revendications 1 à 9, dans lequel au moins une portion de l'arbre (126) allongé avec les un ou plusieurs matériaux antimicrobiens disposés dessus comprend une surface irrégulière et/ou une ou plusieurs cavités.

11. Système selon l'une quelconque des revendications 1 à 10, dans lequel les un ou plusieurs matériaux antimicrobiens sont disposés sur une surface sensiblement entière du dispositif d'administration thérapeutique (100).

12. Système selon l'une quelconque des revendications 1 à 11, dans lequel l'organe (314) de valve comprend un matériau plus déformable et/ou plus résilient que la portion de base (124) de sorte que l'organe (314) de valve est configuré pour épouser le contour d'une surface distale et/ou latérale (120) de la portion de base (124) pour former l'étanchéité.

13. Système selon l'une quelconque des revendications 1 à 12, dans lequel la portion de base (124) est cylindrique.

14. Système selon l'une quelconque des revendications 1 à 13, dans lequel la portion de base (124) présente une section transversale sensiblement uniforme le long d'un axe longitudinal du dispositif d'administration thérapeutique (100).
